# EUROPEAN PATENT APPLICATION

(11) **EP 3 757 573 A1**
(43) Date of publication of application: **30.12.2020**
(21) Application number: 19757269.6
(22) Date of filing: 21.02.2019
(51) Int. Cl.: G01N 33/64, G01N 33/52, G01N 21/78, G01N 33/493

(54) **SPECIMEN FOR DETECTING KETONE BODIES IN URINE**

(30) Priority: 23.02.2018 JP 2018030561
(71) Applicant: SYSMEX CORPORATION, Kobe-shi, Hyogo 651-0073 (JP)
(72) Inventor: ISHIDA, Akiko, Tokyo 110-8408 (JP); OHATA, Kazuo, Tokyo 110-8408 (JP)
(74) Representative: Hoffmann Eitle
(86) International application number: PCT/JP2019/006596
(87) International publication number: WO 2019/163902

(57) **Abstract**

In measurement of ketone bodies in urine, false-positives due to a drug having a thiol group are inhibited. False-positives due to a drug having a thiol group are inhibited by adding an oxidizing agent to a test piece for detection of ketone bodies in urine.

## Description

### TECHNICAL FIELD

The present invention relates to inhibition of non-specific reactions in measurement of ketone bodies in urine.

### BACKGROUND ART

Detection of various components in urine is used for diagnoses of various diseases, in particular, renal disease, urinary tract disease, and diabetes. Collection of a urine specimen hardly causes any burden to a subject, and thus, a urine test is not only performed for patients of hospitals, but also always performed in health examinations.

In general, a urine test piece to be used in a test of a urine specimen has a form in which a plurality of test pieces capable of detecting various components in urine through color reactions are attached to a synthetic resin support body such as PET. The urine test strip is immersed in a urine specimen collected in a paper cup or the like and then is taken out, colorations of test strip portions are visually confirmed, and presence or absence of various components is qualitatively (positive or negative) determined. When the test is performed using a fully automatic analyzer, a urine specimen is dropped on a urine test strip, colorations are optically detected, and the results are compared to a calibration curve, whereby concentrations of various components are calculated.

Examples of items detectable by a urine test strip include glucose, protein, occult blood, and ketone bodies. Other than these, creatinine can also be measured, and the amount of urine protein per day can also be calculated at any time from the protein concentration in urine.

Ketone bodies are a generic term of acetone, acetoacetic acid, and β-hydroxybutyric acid, and usually, ketone bodies are not detected in urine of healthy persons. However, it is known that ketone bodies in urine become positive in the cases of diabetes, hyperthyroidism, and the like.

The detection principle of ketone bodies in urine uses a phenomenon that sodium nitroprusside reacts with acetone or acetoacetic acid to exhibit a purple color. However, sodium nitroprusside also similarly reacts with a compound having a thiol group and exhibits a purple color. That is, when a subject is taking a drug that contains a compound having a thiol group, the drug is discharged into urine and reacts with sodium nitroprusside, whereby the test result of ketone bodies in urine becomes false-positive.

Captopril (antihypertensive drug) and bucillamine (antirheumatic drug) are known as drugs having a thiol group that causes a false-positive of ketone bodies in urine. In particular, since bucillamine could cause proteinuria as a side effect, urine qualitative tests are frequently performed for bucillamine-treated patients. Thus, a false-positive of ketone bodies in urine poses a problem.

### SUMMARY OF THE INVENTION

### PROBLEMS TO BE SOLVED BY THE INVENTION

A problem to be solved by the present invention is to inhibit false-positives due to a drug having a thiol group in measurement of ketone bodies in urine.

### MEANS FOR SOLVING THE PROBLEMS

In order to solve the above problem, the present inventors conducted thorough research and completed the invention indicated below.

The present invention provides a test piece for detection of ketone bodies in urine and a false-positive inhibition method indicated below.
(1) A test piece for detection of ketone bodies in urine, the test piece containing an oxidizing agent.
(2) The test piece for detection of ketone bodies in urine according to (1), wherein the oxidizing agent is an oxidizing agent for inhibiting a false-positive.
(3) The test piece for detection of ketone bodies in urine according to (1) or (2), wherein the oxidizing agent is potassium iodate.
(4) A false-positive inhibition method comprising adding an oxidizing agent in a test piece for detection of ketone bodies in urine.
(5) The false-positive inhibition method according to (4), wherein the oxidizing agent is potassium iodate.

### ADVANTAGEOUS EFFECTS OF THE INVENTION

Since the test piece for detection of ketone bodies in urine of the present invention contains the oxidizing agent, even when a subject of a urine test is taking a drug having a thiol group, a result of the test can be inhibited from becoming false-positive.

### DESCRIPTION OF EMBODIMENTS

A test piece for detection of ketone bodies in urine of the present invention is created as a test piece that contains ketone bodies detection reagent, as described in detail below. Further, a urine test strip for a multi-item test may be formed by providing a test piece for another detection target item on a support body. Alternatively, a test strip for a single item for detecting only ketone bodies in urine may be formed.

The test piece for detection of ketone bodies in urine of the present invention comprises a reagent composition that contains sodium nitroprusside and an oxidizing agent. In the test piece for detection of ketone bodies in urine of the present invention, the oxidizing agent can inhibit sodium nitroprusside from: reacting with a compound having a thiol group; and non-specifically exhibiting coloration to cause a false-positive. The oxidizing agent is not limited in particular, and an alkali metal salt of iodic acid or potassium ferricyanide can be used. Out of these oxidizing agents, potassium iodate is preferable.

The test piece for detection of ketone bodies in urine of the present invention is created by: preparing a solution that contains sodium nitroprusside and an oxidizing agent; causing an absorbent carrier to be impregnated with the solution; and drying the resultant absorbent carrier. The concentration of sodium nitroprusside in the solution is preferably 0.5% to 1.5%. The concentration of the oxidizing agent in the solution is preferably 0.25% to 0.75%.

As the reagent composition to be carried by the test piece, a solubilizer, a sensitizer, a wetting agent, a buffer agent, etc., may be used, in addition to sodium nitroprusside and the oxidizing agent.

Filter paper is often used as the absorbent carrier to be impregnated with the reagent composition. However, cotton, nonwoven fabric, glass fiber, or the like can also be used. Further, an organic polymer such as gelatin or synthetic resin can also be caused to contain the reagent composition.

The test piece for detection of ketone bodies in urine manufactured in this manner is attached to a support body. The test piece for detection of ketone bodies in urine may be attached to the support body together with a test piece that detects another detection target item. For example, a support body in the form of a plastic piece or sheet made from polyvinyl chloride, polyethylene terephthalate, polypropylene, polystyrene, polyester, or the like can be used as the support body.

A test strip obtained in this manner is immersed in a liquid sample that contains urine, and is immediately taken out. Then, coloration of the test piece after a certain time is compared, with eyes, against a previously-created standard color table, whereby a qualitative or semi-quantitative test of the sample is performed. Alternatively, a quantitative test can also be performed by optically measuring reflectance or the like by use of a measurement apparatus, and obtaining a concentration with reference to a calibration curve.

### [Example]

### Example 1

### (Test piece, for detection of ketone bodies in urine, containing oxidizing agent)

A solution, for detection of ketone bodies in urine, that contains 0.8% sodium nitroprusside, 0.4% potassium iodate, and glycine buffer solution (pH 9.0) was prepared. Then, filter paper was caused to be impregnated with the solution and then dried, whereby a test piece for detection of ketone bodies in urine was produced.

### Comparative Example 1

### (Test piece for detection of ketone bodies in urine, for control)

A test piece for detection of ketone bodies in urine, for control, was created in the same manner as that for Example 1, except that the control test piece did not contain potassium iodate.

### Example 2

200 urine specimens were measured, using the test pieces, for detection of ketone bodies in urine, that were created in Example 1 and Comparative Example 1. There were 25 specimens that were negative with the test pieces containing the oxidizing agent but that were positive with the control test pieces. With respect to each of these 25 specimens, ketone bodies measurement result by an enzyme method indicated 0.0 mg/dL (not greater than detection sensitivity). All of these 25 specimens were urine of rheumatoid arthritis patients who were taking bucillamine.

### INDUSTRIAL APPLICABILITY

Since the test piece for detection of ketone bodies in urine of the present invention contains the oxidizing agent, the test piece for detection of ketone bodies in urine can effectively inhibit non-specific reaction due to a compound having a thiol group. Therefore, even when a subject of a urine test is taking a drug having a thiol group, the result of a test of ketone bodies in urine can be inhibited from becoming false-positive.

## Claims

1. A test piece for detection of ketone bodies in urine, the test piece comprising an oxidizing agent.

2. The test piece for detection of ketone bodies in urine according to claim 1, wherein
the oxidizing agent is an oxidizing agent for suppressing a false-positive.

3. The test piece for detection of ketone bodies in urine according to claim 1 or 2, wherein
the oxidizing agent is potassium iodate.

4. A false-positive inhibition method comprising
adding an oxidizing agent in a test piece for detection of ketone bodies in urine.

5. The false-positive inhibition method according to claim 4, wherein
the oxidizing agent is potassium iodate.
